# EUROPÄISCHE PATENTSCHRIFT

(11) **EP 1 263 490 B1**
(45) Veröffentlichungstag und Bekanntmachung des Hinweises auf die Patenterteilung: **27.07.2005**
(21) Anmeldenummer: 01923619.9
(22) Anmeldetag: 02.03.2001
(51) Int. Cl.: A61M 5/30

(54) **NADELLOSER INJEKTOR IN MINIATURAUSFÜHRUNG**
MINIATURIZED NEEDLELESS INJECTOR
INJECTEUR SANS AIGUILLE MINIATURISE

(30) Priorität: 03.03.2000 DE 10010123
(43) Veröffentlichungstag der Anmeldung: 11.12.2002
(73) Patentinhaber: Boehringer Ingelheim International GmbH, 55216 Ingelheim am Rhein (DE)
(72) Erfinder: EICHER, Joachim, 44227 Dortmund (DE); GESER, Johannes, 55218 Ingelheim am Rhein (DE); ZIERENBERG, Bernd, 55411 Bingen am Rhein (DE); REIMHOLZ, Ralph, Christian, 65195 Wiesbaden (DE); ELBERS, Knut, 55435 Gau Algesheim (DE); HENKE, Stefan, 55239 Gau-Odernheim (DE)
(86) Internationale Anmeldenummer: PCT/EP2001/002356
(87) Internationale Veröffentlichungsnummer: WO 2001/064268

(56) Entgegenhaltungen:
- FR-A- 2 629 706
- US-A- 5 964 416

## Beschreibung

Die Erfindung betrifft einen nadellosen Injektor als Handgerät, bevorzugt in Miniaturausführung, mit dem eine Flüssigkeit zum Beispiel in menschliches oder tierisches Gewebe intrakutan injiziert wird. Die Erfindung bezweckt, den Anwendungsbereich eines derartigen Injektors zu erweitern.

Flüssigkeiten im Sinne der vorliegenden Erfindung sind bevorzugt Lösungen, Suspensionen oder Dispersionen, die eine Wirksubstanz enthalten. Wirksubstanzen können pharmakologisch aktive Stoffe zum Behandeln des menschlichen oder tierischen Körpers sein, oder sie können Stoffe zur Diagnose oder kosmetischen Anwendung sein.

Wirkstoffe für nicht-pharmazeutische Anwendung können beispielsweise im Bereich des Pflanzenschutzes Insektizide, Fungizide, wachstumsfördernde oder wachstumshemmende Mittel oder Düngemittel sein. Der erfindungsgemäße nadellose Injektor ermöglicht die umweltfreundliche Anwendung systemisch wirkender Mittel, da der Wirkstoff der Pflanze direkt zugeführt wird.

Aus EP - 0 063 341 und EP - 0 063 342 ist ein nadelloser Injektor bekannt, der eine Kolbenpumpe zum Ausstoßen der zu injizierenden Flüssigkeit enthält, die mit Hilfe eines Druckmittels von einem Motor angetrieben wird. Der Flüssigkeitsbehälter ist seitlich an der Kolbenpumpe angebracht. Die für eine Injektion erforderliche Flüssigkeitsmenge wird beim Zurückziehen des Kolbens über einen Einlaßkanal und eine Rückschlagklappe in die Pumpenkammer gesaugt. Sobald der Kolben in Richtung Düsenkörper bewegt wird, wird die Flüssigkeit durch den Auslaßkanal zur Düse gedrückt und ausgestoßen. Der Kolben der Kolbenpumpe ist ein massiver Rundkolben.

In EP - 0 133 471 wird ein nadelloses Impfgerät beschrieben, das aus einer Siphonpatrone über ein Spezialventil mit unter Druck stehendem Kohlendioxid betrieben wird.

Aus EP - 0 347 190 ist ein Vakuum-Druckgas-Injektor bekannt, bei dem die Eindringtiefe des injizierten Medikaments mittels des Gasdrucks und das Volumen des Medikaments durch den Kolbenhub einstellbar ist.

Aus EP - 0 427 457 ist ein nadelloser hypodermischer Injektor bekannt, der mittels Druckgas über ein zweistufiges Ventil angetrieben wird. Das Injektionsmittel befindet sich in einer Ampulle, die in einen Schutzmantel gesteckt wird, der am Injektorgehäuse befestigt wird. Die Ampulle wird auf das Ende der Kolbenstange aufgesteckt. Am anderen Ende der Ampulle befindet sich die Düse, deren Durchmesser sich zum Ampullenende verjüngt.

In WO - 89/08469 ist ein nadelloser Injektor für einmaligen Gebrauch angegeben. In WO - 92/08508 ist ein nadelloser Injektor angegeben, der für drei Injektionen ausgelegt ist. Die das Medikament enthaltende Ampulle wird in das eine Ende der Antriebseinheit geschraubt, wobei die Kolbenstange in das offene Ende der Ampulle gesteckt wird. Die Ampulle enthält an ihrem einen Ende die Düse, durch die das Medikament ausgestoßen wird. Etwa in der Mitte der Ampullenlänge ist ein verschiebbarer Verschlußstopfen vorhanden. Die zu injizierende Dosis ist über die Einschraubtiefe der Ampulle einstellbar. Die nach einer Betätigung des Injektors aus der Antriebseinheit hervorstehende Kolbenstange wird von Hand zurückgeschoben. Beide Geräte werden mit Druckgas betrieben.

Aus WO - 93/03779 ist ein nadelloser Injektor mit einem zweiteiligen Gehäuse und einem seitlich am Gerät aufgesteckten Flüssigkeitsbehälter bekannt. Die Antriebsfeder für den Kolben wird mittels eines Getriebemotors gespannt. Die Feder wird ausgelöst; sobald die beiden Gehäuseteile durch Andrücken der Düse auf die Injektionsstelle gegeneinander verschoben werden. Jeweils ein Ventil ist im Ansaugkanal für die Flüssigkeit und im Auslaß der Dosierkammer vorhanden.

In WO - 95/03844 ist ein weiterer nadelloser Injektor angegeben. Dieser enthält eine mit Flüssigkeit gefüllte Kartusche, die an einem Ende eine Düse enthält, durch die die Flüssigkeit ausgestoßen wird. Am anderen Ende ist die Kartusche durch einen in die Kartusche einschiebbaren Hutkolben verschlossen. Ein durch eine vorgespannte Feder belasteter Kolben verschiebt nach dem Auslösen der Feder den Hutkolben um ein vorgegebenens Stück in die Kartusche hinein, wobei die zu injizierende Flüssigkeitsmenge ausgestoßen wird. Die Feder wird ausgelöst, sobald die Düse auf die Injektionsstelle hinreichend fest aufgedrückt wird. Dieser Injektor ist für einmaligen oder mehrmaligen Gebrauch vorgesehen. Die Kartusche ist vor dem federbelasteten Kolben angeordnet und ist fester Bestandteil des Injektors. Die Position des Kolbens des für mehrere Anwendungen vorgesehenen Injektors verschiebt sich nach jeder Anwendung ein Stück in Richtung zur Düse. Der Kolben und die Antriebsfeder sind nicht rückstellbar. Die Vorspanung der Feder ist zu Anfang hinreichend groß, um die gesamte Flüssigkeitsmenge in der Kartusche auf einmal auszustoßen. Die Feder kann erst wieder gespannt werden, wenn der Injektor zerlegt wird und das Antriebsteil des Injektors mit einer neuen vollständig gefüllten Kartusche zusammengefügt wird.

In FR - 2 629 706 werden zwei Ausführungsformen eines nadellosen Injektors für zahnmedizinische Verwendung beschrieben, mit denen eine vorgegebene Menge einer Flüssigkeit in das Zahnfleisch injiziert werden kann. Beide Injektoren bestehen jeweils aus zwei Teilen, die koaxial zusammengesteckt werden und miteinander lösbar verbunden sind. Als Arbeitsmedium dient Druckluft, die dem Injektor von außen zugeführt wird. Beim Auslösen des Injektors mittels einer Auslösetaste wird ein Arbeitskolben durch Zusammenspiel von Druckluft, Schraubenfedern und Sperrvorrichtungen schlagartig um ein vorgegebenes Stück verschoben, wobei die zu injizierende Flüssigkeitsmenge durch eine Düse ausgestoßen wird. Gleichzeitig werden mehrere Schraubenfedern gespannt, die mehrere axial verschiebbare Teile innerhalb des Injektors wieder in ihre Ausgangsposition zurückschieben, sobald die angelegte Druckluft nicht mehr wirksam ist. Die zu injizierende Flüssigkeit ist innerhalb des Injektors in einem rohrförmigen steifen Behälter untergebracht, der an seinem einen Ende mit einem innerhalb des Behälters verschiebbaren Stopfen versehen ist, der über einen federbelasteten Kolben die im Behälter vorhandene Flüssigkeit ständig unter Druck hält. Beim Hinausdrücken eines Teils der Flüssigkeit aus dem Behälter in den Flüssigkeitsraum innerhalb des Pumpenzylinders schiebt die Druckfeder über den Kolben den Stopfen ein entsprechendes Stück in den Flüssigkeitsbehälter hinein.

Bei einigen bekannten Ausführungen des nadellosen Injektors ist der Vorratsbehälter für die zu injizierende Flüssigkeit seitlich neben der Antriebseinheit angeordnet. Die zu injizierende Flüssigkeitsmenge wird beim Zurückziehen des massiven Kolbens der Kolbenpumpe in den Pumpenraum gesaugt. Der Einlaßkanal enthält ein Einlaßventil, der Auslaßkanal enthält ein Auslaßventil. Beide Ventile arbeiten mit einer Hilfskraft.

Bei anderen Ausführungen des nadellosen Injektors dient der Vorratsbehälter für die zu injizierende Flüssigkeit direkt als Pumpenkammer und ist dem Kraftstoß ausgesetzt, der beim Ausstoßen der zu injizierenden Flüssigkeitsmenge auftritt.

Bei den mit Druckgas betriebenen nadellosen Injektoren entweicht nach jeder Injektion ein Teil des Druckgases. Der Druckgasbehälter ist gegebenenfalls austauschbar, er ist jedoch nicht unmittelbar wieder mit Druckgas befüllbar. Bei diesen Injektoren ist die Antriebseinheit auszutauschen, sobald der Druckgasbehälter leer ist.

Damit stellt sich die Aufgabe, einen mehrfach verwendbaren nadellosen Injektor einfacher Bauart anzugeben, der bevorzugt für das wiederholte Ausstoßen einer vorgegebenen Flüssigkeitsmenge geeignet ist. Die nach vielen Anwendungen insgesamt ausgestoßene Flüssigkeitsmenge soll bevorzugt größer sein als die in einem Vorratsbehälter enthaltenen Flüssigkeitsmenge. Es soll möglich sein, entweder mehrere Teilmengen der Flüssigkeit aus dem Vorratsbehälter nacheinander zu entnehmen, oder die in einem Vorratsbehälter enthaltene Flüssigkeitsmenge als Ganzes auf einmal zu entnehmen und auszustoßen. Der Vorratsbehälter soll auf einfache Weise austauschbar sein. Der vorgegebenen Flüssigkeitsmenge ist ein hinreichend großer mechanischer Kraftstoß (Impuls) zu erteilen, damit die vorgegebene Flüssigkeitsmenge eine Membran, eine Folie oder ein biologisches Gewebe durchdringt.

Diese Aufgabe wird erfindungsgemäß gelöst durch einen nadellosen Injektor für eine Flüssigkeit, der als Handgerät in einem zylindrischen Gehäuse angeordnet ist und einen Vorratsbehälter für die Flüssigkeit enthält. Das Gehäuse besteht im wesentlichen aus zwei Teilen. Beide Teile sind miteinander lösbar oder unlösbar verbunden und gegeneinander drehbar angeordnet. Der nadellose Injektor enthält ein Sperrspannwerk für einen federbetätigten Abtrieb, das vor dem Ausstoßen einer vorgegebenen Menge der Flüssigkeit gespannt wird, und das mit einer Auslösevorrichtung versehen ist. Im Sprungstück des Sperrspannwerks ist ein Hohlkolben befestigt, der durch das Sperrspannwerk angetrieben wird. Der Hohlkolben ist innerhalb eines Zylinders verschiebbar angeordnet. Er ragt mit seinem einen Ende aus dem Zylinder heraus. Bevorzugt an seinem anderen Ende ist ein Ventilkörper angebracht, der der einzige Ventilkörper des nadellosen Injektors ist. Am Ende des Zylinders ist eine Düse mit mindestens einer Öffnung angebracht. Der Raum zwischen der Düse und dem Ende des Hohlkolbens ist der Pumpenraum. Innerhalb des Gehäuses ist ein Vorratsbehälter für die Flüssigkeit vorhanden. Der Vorratsbehälter ist als ein vom nadellosen Injektor separater Behälter ausgebildet, der - bevorzugt mittels einer Preßpassung - mit dem Ende des Hohlkolbens lösbar verbunden ist, das aus dem Zylinder herausragt. Die vorgegebene Menge der Flüssigkeit, die beim Zurückbewegen des Sprungstückes und des damit verbundenen Hohlkolbens durch den Hohlkolben hindurch in den Pumpenraum gefördert worden ist, wird durch den Hub und den Querschnitt des Hohlkolbens bestimmt.

Das Sperrspannwerk besteht aus einem federbelasteten Abtriebsflansch als Sprungstück, einem Antrieb zum Spannen der Feder, einem Sperrglied, zwei Anschlägen für den Abtriebsflansch, zwischen denen sich der Abtriebsflansch hin und her bewegen kann, und einer Vorrichtung zum Auslösen des Sperrgliedes. Der Weg des Abtriebsteils ist durch die beiden Anschläge präzise begrenzt. Zwischen der energiespeichernden Feder und dem Antrieb zum Spannen der Feder ist ein kraftübertragendes Getriebe angeordnet. Das Sperrglied ist ringförmig und hat einrückende Sperrflächen. Als Energiespeicher kann eine bevorzugt zylindrische Schraubenfeder oder eine Tellerfeder oder eine Blattfeder verwendet werden, die als Zugfeder oder als Druckfeder wirkt.

Die energiespeichernde Feder kann mittels eines Direktantriebes gespannt werden. Dazu wird der Abtriebsflansch durch eine axial wirkende äußere Kraft verschoben. Bei großer Federkraft ist ein kraftübersetzendes Getriebe vorteilhaft, zum Beispiel ein Schraub-Schub-Getriebe, mittels dem die Feder durch ein äußeres Drehmoment gespannt wird. Ein derartiges Getriebe ist ein ein- oder mehrgängiges Getriebe, das zwischen der Feder und dem Antrieb zum Spannen der Feder angeordnet ist.

Der Abtriebsflansch kann topfförmig ausgebildet sein. Der Kragen des Abtriebsflansches kann beispielsweise zwei sägezahnförmige Aussparungen enthalten, auf denen zwei Sägezähne im oberen Gehäuseteil abgleiten.

Die mittlere Federkraft kann 10 N bis 150 N betragen. Zwischen den beiden Positionen des Sprungstückes des Sperrspannwerkes ändert sich die Federkraft etwa um ± 10 % der mittleren Federkraft.

Das Sperrglied kann ein in sich radial elastisch verformbarer Ring sein, oder ein starrer Ring mit Verschiebenocken, oder ein starrer Ring mit angeformten Blattfedern, oder ein Ring, der durch eine oder mehrere Metallfedern unter Vorspannung gesetzt wird. Der Ring kann geschlossen oder offen sein; er kann aus mehreren Teilen bestehen. Das Sperrglied ist in einer Ebene senkrecht zur Gehäuseachse verschiebbar angeordnet, oder es ist in dieser Ebene verformbar.

Weitere Einzelheiten zu dem Sperrspannwerk für einen federbetätigten Abtrieb sind in DE - 195 45 226 beschrieben.

Im Sprungstück des Sperrspannwerks ist ein Hohlkolben befestigt, der durch das Sperrspannwerk angetrieben wird. Der Hohlkolben taucht in den Zylinder ein und ragt mit einem Teil seiner Länge aus dem Zylinder heraus; er ist innerhalb des Zylinders verschiebbar angeordnet.

Am Ende des Zylinders ist eine Düse angebracht. Die Düsenöffnung kann einen hydraulischen Durchmesser von 10 µm bis 500 µm, bevorzugt von 50 µm bis 150 µm, haben. Die Düsenöffnung kann eine Länge von 50 µm bis 500 µm, bevorzugt von 100 µm bis 300 µm haben.

Bei mehreren Öffnungen in der Düse können die Längsachsen der Düsenöffnungen zueinander parallel verlaufen, oder sie können divergent gegeneinander geneigt sein. Bei mehreren Düsenöffnungen kann deren hydraulischer Durchmesser unterschiedlich sein.

Die Düse kann ein aus zwei Siliziumplatten zusammengesetzter Quader sein, der zum Beispiel 1,1 mm breit, 1,5 mm lang und 2,0 mm hoch ist. Der Quader kann in der Berührungsfläche der Platten eine flache dreieckförmige etwa 400 µm dicke Aussparung enthalten, die in einer einzigen Düsenöffnung endet, die 50 µm breit, 50 µm dick und 200 µm lang ist. Es kann zweckmäßig sein, die Düse auf ihrem ganzen Umfang mit einem paßgenauen elastomeren Formteil zu umgeben. Die Innenkontur des elastomeren Formteils ist an die Außenkontur der Düse angepaßt, die Außenkontur des elastomeren Formteils ist an die Innenkontur eines bevorzugt aus Metall bestehenden Düsenhalters angepaßt. Eine derartige "schwimmende Halterung" macht die Düse aus sprödem Material unempfindlich gegen stoßartig wirkende Belastungen, die bei bestimmungsgemäßem Gebrauch des nadellosen Injektors auftreten.

Bevorzugt an dem Ende des Hohlkolbens, das sich innerhalb des Zylinders befindet, ist ein bevorzugt aus einem Stück bestehender Ventilkörper angebracht, der durch den Hohlkolben geführt wird, und der gegen den Hohlkolben axial verschiebbar angeordnet ist. Der Ventilkörper bewegt sich im wesentlichen mit dem Hohlkolben. Der Ventilkörper hat bevorzugt eine einachsig rotationssymmetrische Form, wie zum Beispiel ein Kreiszylinder oder ein Kegelstumpf. Sein Durchmesser kann kleiner sein als der Durchmesser des Raumes, in dem der Ventilkörper verschiebbar angeordnet ist. Der Ventilkörper kann sich um seine Achse drehen. Die Achse des Ventilkörpers bleibt stets parallel zur Achse des Hohlkolbens. Damit liegt eine definierte Dichtfläche auf der Einlaßseite des Ventilkörpers vor. Der Weg, auf dem sich der Ventilkörper relativ zum Hohlkolben verschieben kann, ist durch einen Anschlag begrenzt. In der Position, in der der Ventilkörper an der definierten Dichtfläche anliegt, ist das Ventil geschlossen.

Der Raum zwischen der Düse und dem am Hohlkolben angebrachten Ventilkörper ist der Pumpenraum. Vor dem düsenseitigen Ende des Pumpenraumes, also im Ausstoßkanal für die Flüssigkeit, kann ein Filter angebracht sein, das bevorzugt als Tiefenfilter ausgebildet ist. Falls die zu injizierende Flüssigkeit suspendierte Teilchen enthält, ist die Porenweite des Filters an die Teilchengröße anzupassen.

Weitere Einzelheiten zum Hohlkolben und zum Ventilkörper sind in DE - 195 36 902 angegeben.

Das Sperrspannwerk und die energiespeichernde Feder werden bevorzugt durch Drehen der beiden Gehäuseteile gegeneinander, bevorzugt über ein Schraub-Schub-Getriebe gespannt. Das Drehmoment kann von Hand oder mittels eines Motors erzeugt werden.

Der Zylinderdurchmesser stimmt bevorzugt auf seiner ganzen Länge mit dem Außendurchmesser des Hohlkolbens praktisch überein. Der Zylinder kann in dem einen Gehäuseteil fest angebracht sein. Weiter kann der Zylinder in dem einen Gehäuseteil axial verschiebbar angebracht sein. Der verschiebbare Zylinder wird durch eine Rückstellfeder in seiner Ruhelage gehalten.

Die beiden Anschläge für das Sprungstück können im Gehäuse fest positioniert sein. Weiter kann die Position eines dieser Anschläge in axialer Richtung veränderbar sein. Damit kann das Volumen des Pumpenraumes bei konstantem Außendurchmesser des Hohlkolbens verändert werden. Bei sonst unverändertem Aufbau des nadellosen Injektors kann die Menge der ausgestoßenen Flüssigkeit durch Verändern der Position eines Anschlages verändert werden.

Die Lage des Weges des Sprungstücks und damit der Hub des Hohlkolbens innerhalb des nadellosen Injektors wird durch die beiden Anschläge begrenzt. Bei gegebener Position der Anschläge ist die Lage des Weges des Sprungstückes und damit der Hub des Hohlkolbens bei jeder Injektion konstant.

Mittels einer Auslösevorrichtung wird das Sperrglied parallel zur Ringebene verschoben oder in der Ringebene radial verformt. Bei im Gehäuse fest angebrachtem Zylinder wird die Auslösevorrichtung mittels einer mit einem Finger eindrückbaren Auslösetaste betätigt und das Sperrglied ausgelöst. Bei im Gehäuse verschiebbar angebrachtem Zylinder wird die Auslösevorrichtung beim Hineindrücken des Zylinders gegen die Kraft der Rückstellfeder betätigt und das Sperrglied ausgerückt.

Innerhalb des Gehäuses ist ein Vorratsbehälter für die Flüssigkeit vorhanden. Dieser Vorratsbehälter ist als vom nadellosen Injektor separater Behälter ausgebildet; er ist mit dem Ende des Hohlkolbens verbunden, das dem Pumpenraum gegenüber liegt. Das Ende des Hohlkolbens ist von der im Vorratsbehälter befindlichen Flüssigkeit bedeckt.

Der mit dem Hohlkolben verbundene Vorratsbehälter kann zusätzlich mit dem Sprungstück verbunden sein. Diese Verbindung kann eine lösbare oder unlösbare Steckverbindung sein, bei der das Sprungstück mit mehreren Schnapphaken versehen ist, die in eine umlaufende Rille im Vorratsbehälter eingreifen, nachdem der Vorratsbehälter in den nadellosen Injektor eingeschoben worden ist.

Die vorgegebene Menge der auszustoßenden Flüssigkeit wird durch den Hub und den Querschnitt des Hohlkolbens bestimmt. Der Hub des Hohlkolbens ist durch die beiden Anschläge für den Abtriebsflansch begrenzt.

Es ist zweckmäßig, vor der Düse eine abnehmbare Verschlußkappe zum Schutz der Düsenöffnung während der Lagerung des nadellosen Injektors vor und während dessen Gebrauchsdauer gegen Verschmutzung und Verdunstung der Flüssigkeit vorzusehen.

Die beiden Teile des Gehäuses, das Sperrspannwerk, der Zylinder und der Vorratsbehälter bestehen bevorzugt aus Kunststoff, zum Beispiel aus Polybutylenterephthalat. Der Hohlkolben besteht bevorzugt aus Metall, zum Beispiel aus Edelstahl.

Der Ventilkörper kann aus Metall, Keramik, Glas, Edelstein, Kunststoff oder Elastomer bestehen.

Die Düse kann aus Metall, Kunststoff, Glas, Silizium oder Edelstein, wie Saphir, Rubin, Korund, bestehen.

Das Filter besteht bevorzugt aus gesintertem Metall oder gesintertem Kunststoff.

Der nadellose Injektor wird bevorzugt als Handgerät gefertigt. Er kann bei der Injektion mit einer Hand gehalten und bedient werden. Der Zylinder, der Hohlkolben, der Ventilkörper, die Düse und gegebenenfalls das Filter sind miniaturisierte Bauteile.

Im folgenden wird die Funktionsweise des nadellosen Injektors beschrieben.

Während der Lagerung des unbenutzten oder des bereits benutzten nadellosen Injektors sowie zwischen zwei Injektionen befindet sich der nadellose Injektor im Ruhezustand. Die energiespeichernde Feder steht unter Vorspannung. Das Sprungstück liegt an dem Anschlag an, der den Weg des Sprungstücks im Ruhezustand begrenzt. Der Hohlkolben taucht tief in den Zylinder ein. Zwischen dem Ende des Hohlkolbens und der Innenseite der Düse ist ein nur geringer Abstand vorhanden. Das Sperrglied befindet sich in ausgerückter Position.

Beim Drehen der beiden Gehäuseteile gegeneinander wird das Sperrspannwerk gespannt. Das Sprungstück wird in axialer Richtung vom Zylinder weg verschoben, wobei die Spannung der energiespeichernden Feder erhöht wird. Gleichzeitig wird der Hohlkolben ein Stück aus dem Zylinder herausgezogen, und der Pumpenraum wird vergrößert. Der Hohlkolben ragt mit einem Teil seiner Länge weiterhin in den Zylinder hinein. Gleichzeitig wird ein Teil der im Vorratsbehälter enthaltenen Flüssigkeit durch den Hohlkolben hindurch und am Ventilkörper vorbei in den Pumpenraum gefördert, und der Pumpenraum wird mit Flüssigkeit gefüllt. Die Menge der Flüssigkeit im Pumpenraum stimmt mit der bei einer Injektion ausgestoßenen Menge der Flüssigkeit praktisch überein. Das Sprungstück wird so weit verschoben, bis das Sperrglied in seine eingerückte Position springt. Bei einem nadellosen Injektor mit Auslösetaste tritt diese Taste etwas aus dem Gehäuse heraus.

Das düsenseitige Ende des nadellosen Injektors wird auf die Injektionsstelle aufgesetzt und angedrückt. Bei einem nadellosen Injektor mit Auslösetaste wird die Auslösetaste mit einem Finger betätigt und in das Gehäuse hineingedrückt. Dadurch wird das Sperrglied in die ausgerückte Position geschoben, und die Injektion wird ausgelöst. Bei einem nadellosen Injektor mit verschiebbar angeordnetem Zylinder wird der Injektor mit seinem düsenseitigen Ende von Hand mit zunehmender Kraft gegen die Kraft der Rückstellfeder auf die Injektionsstelle gedrückt. Dabei wird der Zylinder in das Gehäuse hineingeschoben, das Sperrglied wird in seine ausgerückte Position geschoben, und die Injektion wird ausgelöst. Beim Abheben des nadellosen Injektors von der Injektionsstelle drückt die Rückstellfeder den Zylinder in seine Ruhelage zurück.

Sobald das Sperrglied die ausgerückte Position eingenommen hat, wirkt die Kraft K der gespannten energiespeichernden Feder über das Sprungstück, den Hohlkolben und das geschlossene Ventil am Ende des Hohlkolbens während des Zeitabschnitts Δt auf die im Pumpenraum befindliche Flüssigkeit, wodurch die Flüssigkeitsmasse m die Geschwindigkeit Δv und damit einen mechanischen Impuls K · Δt = m · Δv erhält, mit großer Geschwindigkeit aus der Düse austritt und in das Gewebe intrakutan eindringt. Nach der Injektion befindet sich der nadellose Injektor wieder im Ruhezustand.

Der erfindungsgemäße nadellose Injektor kann in der Humanmedizin und in der Tiermedizin zum intrakutanen Injizieren einer als Flüssigkeit vorliegenden Zubereitung eines Wirkstoffs, zum Beispiel eines Arzneimittels, in menschliches oder tierisches Gewebe dienen. Beispiele für geeignete pharmazeutische Zubereitungen sind unter anderem Analgetika, Impfstoffe, Antidiabetika, Hormone, Mittel zur Empfängnisverhütung, Vitamine, Antibiotika, Sedativa, antimikrobielle Substanzen, Aminosäuren, Koronarmittel.

Die Zubereitung des Arzneimittels kann in Form einer Lösung, einer Suspension oder einer Emulsion vorliegen. Bei Suspensionen sollte die mittlere Teilchengröße 15 µm, bevorzugt 10 µm, nicht überschreiten.

Geeignete Mittel zum Lösen, Suspendieren oder Emulgieren von Wirkstoffen und gegebenenfalls erforderlichen Hilfsstoffen sind beispielsweise Wasser, Alkohole, Alkohol-Wasser-Mischungen sowie Emulsionen von Öl in Wasser oder Wasser in Öl. Dazu gehören gereinigtes, sterilisiertes Wasser, Ethanol, Propandiol, Benzylalkohole, Ethanol-Wasser-Gemische, Öle (wie Kokosnussöl, Erdnussöl, Sojaöl, Rizinusöl, Sonnenblumenöl), Fettsäureester (wie Isopropylmyristat, Isopropylpalmitat, Ethyloleat), Triglyceride, Triacetin, Solketal, Propylenglykol. Weiter können die Formulierungen Hilfsstoffe wie beispielsweise Konservierungsmittel sowie Säuren oder Basen zum Einstellen des pH-Wertes enthalten.

Die vorgegebene Menge einer Flüssigkeit kann mittels des nadellosen Injektors in ein Blatt oder den Stengel einer Pflanze oder durch eine Membran hindurch in den Raum hinter der Membran injiziert werden.

Der erfindungsgemäße nadellose Injektor hat folgende Vorteile:
- Er hat eine handliche Form. Der Vorratsbehälter für die Flüssigkeit befindet sich im Injektorgehäuse.
- Er ist für viele - bis zu mehreren hundert - Injektionen verwendbar, die aus einem oder aus mehreren Vorratsbehältern entnommen werden können.
- Er hat außer dem Ventil am Ende des Hohlkolbens keine weiteren Ventile.
- Das Sperrspannwerk läßt sich selbst bei großen Federkräften auch von ungeübten Personen einfach handhaben und ist über ein Schraub-Schub-Getriebe mit relativ geringem Kraftaufwand zu spannen.
- Das Sperrspannwerk wird von Hand durch Drücken der Auslösetaste mit einem Finger oder beim Andrücken des nadellosen Injektors auf die Injektionsstelle ausgelöst.
- Die Antriebseinheit wird nicht ausgetauscht, sondern nur der Vorratsbehälter für die Flüssigkeit.
- Das am Ende der Hohlkolbens angebrachte Ventil arbeitet ohne Hilfskraft und schließt sehr schnell.
- Das Volumen des Pumpenraumes ist durch Verändern der Position eines der beiden Anschläge veränderbar.
- Der mechanische Impuls der zu injizierenden Flüssigkeitsmenge kann an die gewünschte Eindringtiefe in das Gewebe oder an die Dicke der zu durchdringenden Membran angepaßt werden.
- Der Vorratsbehälter für die Flüssigkeit ist an die Bedingungen bei - gegebenenfalls jahrelanger - Lagerung des Behälters angepaßt sowie an dessen Anschluß an den Injektor.
   Seine Auslegung ist unabhängig von den Forderungen, die an die Pumpenkammer vor der Düse gestellt werden.
- Der Vorratsbehälter für die Flüssigkeit ist dem Kraftstoß beim Injizieren nicht ausgesetzt.
- Der Vorratsbehälter für die Flüssigkeit ist auf einfache Weise austauschbar.
- Die für einen vorgegebenen Anwendungsfall erforderliche Menge der zu verabreichenden Flüssigkeit kann auf einfache Weise in mehreren Teilmengen nacheinander an verschiedenen Stellen des Injektionsbereiches injiziert werden.
- Die nadellose Injektion beeinträchtigt die Injektionsstelle wesentlich weniger als die Injektion mittels einer Injektionsspritze.

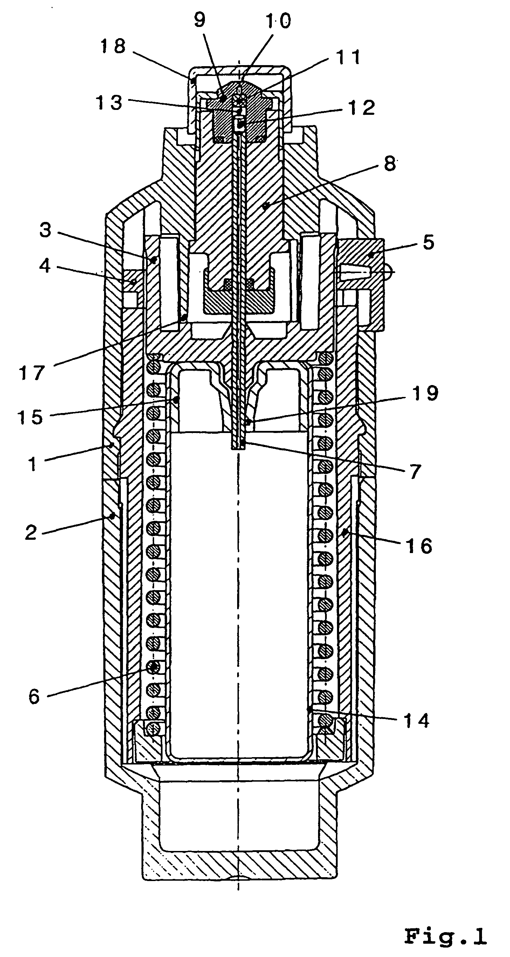

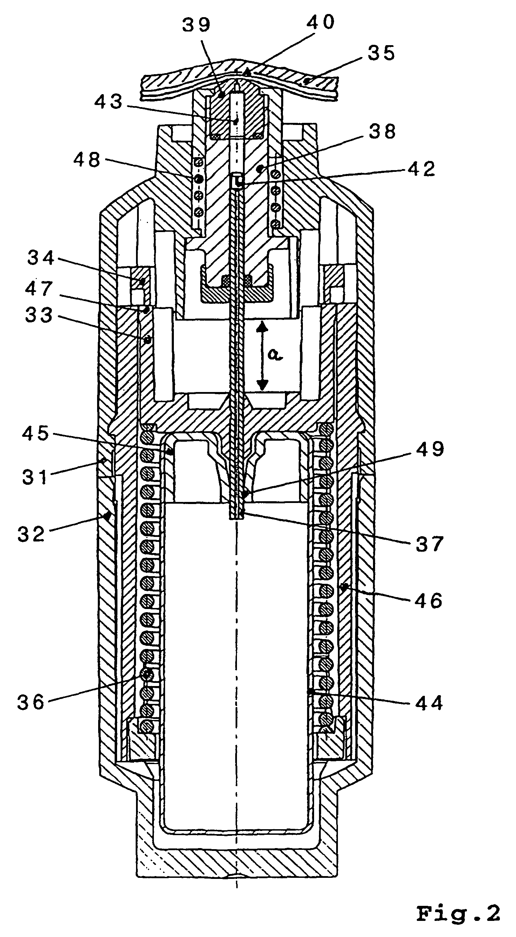

Die Erfindung wird an Hand der Figuren näher erläutert. In Figur 1 ist der Längsschnitt durch einen nadellosen Injektor mit Auslösetaste im Ruhezustand dargestellt, bei dem der Zylinder in dem einen Gehäuseteil fest angeordnet ist. Figur 2 zeigt den Längsschnitt durch einen nadellosen Injektor ohne Auslösetaste im gespannten Zustand der energiespeichernden Feder, bei dem der Zylinder in dem einen Gehäuseteil verschiebbar angeordnet ist.

In Figur 1 sind die beiden Gehäuseteile (1) und (2) dargestellt, die miteinander lösbar verbunden und gegeneinander drehbar angeordnet sind. Von dem im Ruhezustand befindlichen Sperrspannwerk sind dargestellt das Sprungstück (3), das Sperrglied (4) im ausgerückten Zustand, die auf das Sperrglied wirkende Auslösetaste (5) und die energiespeichernde Schraubenfeder (6) in Form einer Druckfeder. Im Sprungstück (3) ist der Hohlkolben (7) befestigt, der in den Zylinder (8) eintaucht. Am Ende des Zylinders ist die Düse (9) mit der Düsenöffnung (10) angebracht. Vor der Düse liegt das Filter (11). Das düsenseitige Ende des Hohlkolbens ist mit dem Ventilkörper (12) versehen. Zwischen Ventilkörper und Filter befindet sich der Pumpenraum (13). Der Vorratsbehälter (14) ist in dem sonst freien Raum innerhalb der Schraubenfeder angeordnet; er ist im Flansch (15) auf den Hohlkolben aufgesteckt und wird auf dem Hohlkolben durch die Preßpassung (19) gehalten. Der Käfig (16), der die Schraubenfeder umgibt, ist mit dem Gehäuseteil (1) formschlüssig verbunden. Das Sprungstück (3) liegt am Anschlag (17) an. Die Düse ist durch die abnehmbare Verschlußkappe (18) geschützt.

In Figur 2 sind die beiden Gehäuseteile (31) und (32) dargestellt, die miteinander lösbar verbunden und gegeneinander drehbar angeordnet sind. Von dem Sperrspannwerk im gespannten Zustand sind dargestellt das Sprungstück (33), das Sperrglied (34) im eingerückten Zustand und die energiespeichernde Schraubenfeder (36) im gespannten Zustand. Im Sprungstück (33) ist der Hohlkolben (37) befestigt, der in den Zylinder (38) eintaucht. Am Ende des Zylinders ist die Düse (39) mit der Düsenöffnung (40) angebracht. Das düsenseitige Ende des Hohlkolbens ist mit dem Ventilkörper (42) versehen. Zwischen Ventilkörper und Düse befindet sich der Pumpenraum (43). Der Vorratsbehälter (44) ist in dem sonst freien Raum innerhalb der Schraubenfeder angeordnet; er ist im Flansch (45) auf den Hohlkolben aufgesteckt und wird auf dem Hohlkolben durch die Preßpassung (49) gehalten. Der Käfig (46), der die Schraubenfeder umgibt, ist mit dem Gehäuseteil (31) formschlüssig verbunden. Das Sprungstück (33) liegt am eingerückten Sperrglied (34) am Anschlag (47) an. Der Zylinder (38) ist im Gehäuseteil (31) axial verschiebbar angeordnet; er wird durch die schraubenförmige Rückstellfeder (48), die als Druckfeder wirkt, in seiner Ruhelage gehalten. Der Zylinder (38) ist mit einer nicht dargestellten Auslösevorrichtung versehen, die das Sperrglied (34) ausrückt, sobald der Zylinder (38) gegen die Kraft der Rückstellfeder beim Aufdrücken des nadellosen Injektors auf die Injektionsstelle in das Gehäuseteil (31) hineingeschoben wird. Mit (a) ist der Weg des Abtriebsteils zwischen den beiden Anschlägen angedeutet. Der Hub des Hohlkolbens stimmt mit diesem Weg überein.

Die Figur 2 zeigt den zur Injektion angesetzten nadellosen Injektor. Die Düse (39) ist auf die schematisch dargestellte straff gespannte Haut (35) gedrückt; bei weiterem Andrücken auf die Injektionsstelle wird der nadellose Injektor ausgelöst, und die Flüssigkeit wird aus dem Pumpenraum (43) in die Haut (39) injiziert.

Die Figuren 3 und 4 zeigen das eine Ende des Vorratsbehälters und das Sprungstück in einer weiteren Ausführungsform. In Figur 3 ist der Hohlkolben in den Vorratsbehälter eingeführt, jedoch noch nicht mit dem Hohlkolben verbunden.

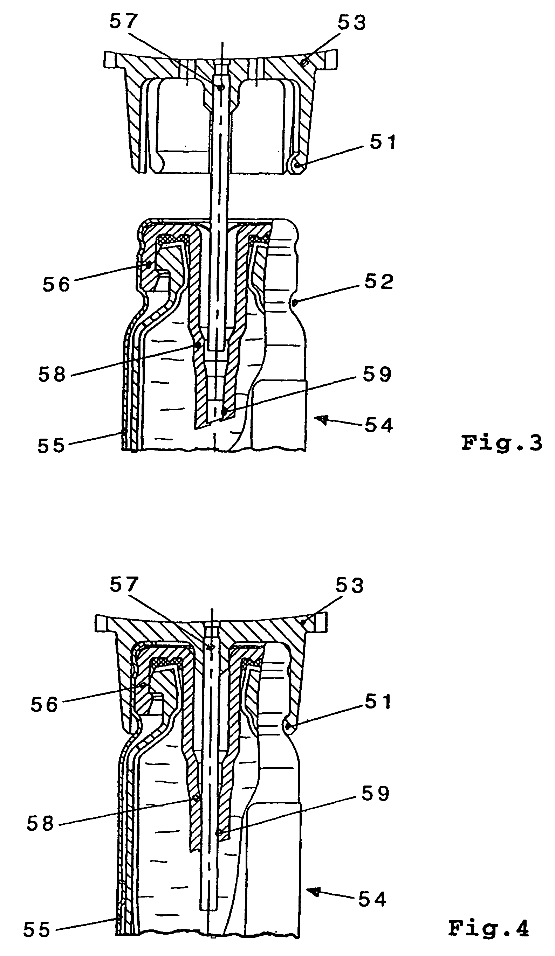

In Figur 3 ist der (dreischalige) Vorratsbehälter (54) teilweise im Längsschnitt dargestellt. Die äußere Schale des Vorratsbehälters ist eine steife Hülse (55), die mit einer umlaufenden Rille (52) versehen ist. Der Vorratsbehälter ist mit dem Stopfen (56) verschlossen, der in den Eintauchstutzen (58) mit Preßpassung (59) übergeht. Ein Abschnitt des Sprungstücks (53) mit dem darin befestigten Hohlkolben (57) ist im Längsschnitt dargestellt. Das Sprungstück ist auf seiner dem Vorratsbehälter zugewandten Seite mit mehreren Schnapphaken (51) versehen.

In Figur 4 ist der Vorratsbehälter mit dem Hohlkolben und dem Sprungstück verbunden, und zwar mit dem Hohlkolben durch die Preßpassung (59) und mit dem Sprungstück über die Schnapphaken (51), die in die umlaufende Rille (52) des Vorratsbehälters eingreifen.

Die in Figur 3 und in Figur 4 dargestellte Verbindung zwischen Vorratsbehälter und Sprungstück besteht aus Schnapphaken (51) mit runden Schultern und einer umlaufenden Rille (52) mit halbkreisförmigem Querschnitt. Diese Verbindung ist eine lösbare Steckverbindung.

Für eine unlösbare Steckverbindung können Schnapphaken mit sägezahnförmigen Schultern und eine umlaufende Rille mit dreieckförmigem Querschnitt gewählt werden.

### Beispiel 1: Aufbau eines erfindungsgemäßen nadellosen Injektors

Ein nadelloser Injektor für die intrakutane Injektion in biologisches Gewebe hat folgende Merkmale:

Das Gehäuse hat einen Außendurchmesser von etwa 20 mm und eine Länge von etwa 70 mm. Beide Gehäuseteile, das Sperrspannwerk und der Federkäfig sind aus Polybutylenterephthalat gefertigt. Der Zylinder besteht ebenfalls aus Polybutylenterephthalat; er hat einen Außendurchmesser von 5 mm und einen Innendurchmesser von 1,60 mm. Die Düse besteht aus Quarz. Die Düsenöffnung hat einen Durchmesser von 140 µm und eine Länge von 220 µm. Der Hohlkolben aus Edelstahl hat einen Außendurchmesser von 1,59 mm und einen Innendurchmesser von 0,35 mm. Der Kolbenhub beträgt 12 mm. Der Ventilkörper besteht aus Elastomer; er hat die Form einer 2 mm dicken Scheibe mit einem Außendurchmesser von 1,60 mm. Die Scheibe ist auf ihrer Mantelfläche mit axialen Aussparungen versehen, durch die die Flüssigkeit am Ventilkörper vorbei in den Pumpenraum strömen kann. Das Ende des Hohlkolbens ist mit einer Nut versehen, in die der Ventilkörper eingreift. Die ausgestoßene Flüssigkeitsmenge beträgt etwa 23 mm³. Der austauschbare Vorratsbehälter hat ein Volumen von etwa 11 cm³.

### Beispiel 2: Intrakutane Applikation einer Flüssigkeit

Eine Injektionslösung aus 20 g Dextran-Fluorescein (UW 3000) pro Liter destilliertes Wasser wurde an zwei narkotisierten Hunden unter Verwendung des erfindungsgemäßen nadellosen Injektors durch die Haut injiziert. Dazu wurden 4,5 ml der Dextran-Fluorescein-Lösung in den Vorratsbehälter des nadellosen Injektors gefüllt, und der Vorratsbehälter wurde an den Hohlkolben des Injektors angeschlossen. Der Injektor wurde durch mehrmaliges Spannen und Auslösen des Sperrspannwerkes betätigt, um die Luft aus dem Hohlkolben, der Pumpenkammer und der Düse herauszudrücken. Anschließend wurde der nadellose Injektor auf eine zuvor rasierte Hautstelle im Bereich des Bauches der Hunde aufgesetzt und ausgelöst. Dieser Vorgang wurde mehrfach wiederholt.

In regelmäßigen Abständen wurden den Hunden Blutproben entnommen, und der Gehalt an Dextran-Fluorescein im Blutplasma wurde bestimmt. Die Ergebnisse belegen die Funktionsfähigkeit des erfindungsgemäßen nadellosen Injektors.

### Beispiel 3: In-vitro-Untersuchung einer Viren-Suspension

Bei Laboruntersuchungen unter Benutzung des nadellosen Injektors wurde ermittelt, ob die Lebensfähigkeit von suspendierten Lebend-Viren herabgesetzt wird, wenn die Suspension durch die Düse des nadellosen Injektors ausgestoßen wird.

An der nach dem Ausstoßen aus dem nadellosen Injektor aufgefangenen Virensuspension wurde eine Abnahme um nur etwa 1 log₁₀ PFU (plaque forming units) bei relativ großen DNA-Viren (Testvirus: Vaccinia virus) und um weniger (um etwa 0,5 log₁₀ PFU) bei kleinen RNA-Viren (Testvirus: Bovine viral diarrhea virus) festgestellt.

### Beispiel 4: In-vivo-Applikation eines Impfstoffs mit modifizierten Lebend-Viren

In einem Tierversuch wurde die Verwendbarkeit des nadellosen Injektors zur Verabreichung einer Impfstoff-Suspension mit modifizierten Lebend-Viren untersucht. Bei dieser Untersuchung wurde sowohl die Sicherheit und die Verträglichkeit einer Impfung mittels des nadellosen Injektors als auch die Wirksamkeit dieses Verabreichungsweges ermittelt.

Sechs gesunde gleichaltrige Hunde wurden zwei Gruppen zugeordnet. Gruppe 1 umfasste zwei Hunde, Gruppe 2 umfasste vier Hunde. Die Tiere beider Gruppen wurden in einem zeitlichen Abstand von jeweils drei Wochen jeweils dreimal mit einem modifizierten Lebend-Impfstoff des Hunde-Adenovirus (canine adeno virus) geimpft.

In Gruppe 1 wurde jeweils 1 Milliliter des Hunde-Adenovirus-Impfstoffs (CAV-1) (Galaxy DA2ppvL+Cv, SNo 610041; Solvay Animal Health Inc.) intramuskulär gemäß den Empfehlungen des Herstellers mittels einer Injektionsspritze verabreicht. In Gruppe 2 wurde ein experimenteller Hunde-Adenovirus-Impfstoff mittels des nadellosen Injektors verabreicht.

Der für die Impfung der Tiere in Gruppe 2 benutzte experimentelle Impfstoff (CAV-2) wurde aus einem abgeschwächten Stamm des Hunde-Adenovirus hergestellt. Der Titer betrug 7,2 log₁₀ TCID₅₀ pro 60 Mikroliter (TCID = tissue culture infective dose). Zu jedem Impfzeitpunkt wurden sechs Einzelschüsse verabreicht (sechsmal 10 Mikroliter = 60 Mikroliter für jede Impfung). Der Injektionsbereich auf dem Rücken der Hunde wurde rasiert, und mittels eines Kugelschreibers wurden die jeweils sechs Injektionsstellen markiert.

Die Wirksamkeit der Impfung wurde durch Bestimmung der Anzahl Virusneutralisierender Antikörper im Serum der Hunde jeweils drei Wochen nach jeder Impfung ermittelt.

Die Verträglichkeit wurde durch Beobachtung der Injektionsstellen sechs Stunden nach der Impfung und anschließend täglich bis zum Abschluß des Tierversuches ermittelt. Die Injektionsbereiche wurden photographiert, und der Befund nach Abtasten der Injektionsstellen wurde notiert.

Diese Versuch hat gezeigt:

Bei den Tieren in Gruppe 2 war eine sehr geringe Rötung der Injektionsstellen während der ersten 2 bis 3 Tage zu erkennen. Eine 1 bis 2 Tage dauernde vorübergehende Schwellung konnte nur durch Abtasten festgestellt werden. Diese geringe und durchaus annehmbare örtliche Reaktion hängt sehr wahrscheinlich mit der örtlichen Amplifizierung des verabreichten modifizierten Lebend-Virus zusammen und ist deshalb als notwendig anzusehen für die gute Wirksamkeit des Impfstoffs. Diese Erklärung wird unterstützt durch die Tatsache, daß nach der Injektion von physiologischer Kochsalzlösung unter Benutzung des nadellosen Injektors gar keine Färbung und keine auch nur geringe vorübergehende Schwellung zu bemerken war.

Die Wirksamkeit wurde durch den Virus-Neutralisations-Test ermittelt. Die Ergebnisse sind in Tabelle 1 angegeben. Die größten Titer sind noch in der Lage, Hunde-Adenoviren zu neutralisieren.

Im Serum aller Tiere aus beiden Gruppen wurden Virus-neutralisierende Antikörper drei Wochen nach der ersten Impfung nachgewiesen. Nach der zweiten und dritten Impfung zeigte sich ein kleiner Verstärkungseffekt.

Die Impfung mit dem experimentellen Hunde-Adenovirus-Impfstoff mittels des nadellosen Injektors ist genau so wirksam wie die intramuskuläre Impfung mit dem handelsüblichen Impfstoff mittels einer Injektionsspritze.

**Tabelle 1:**

| Hunde-Adenovirus; Virus-Neutralisationstiter von Hundeserum | | | | |
|---|---|---|---|---|
| Hund | | VN-Titer von Hundeserum | | |
| Versuchsnummer | Kennzeichen | 21 Tage nach der ersten Impfung | 21 Tage nach der zweiten Impfung | 21 Tage nach der dritten Impfung |
| CAV-1 Handelsüblicher Impfstoff intramuskulär mittels Injektionsspritze | | | | |
| 1496 | -------- | 1024 | 1024 | 4096 |
| 1498 | -------- | 1024 | 1024 | 512 |

| CAV-2 Experimenteller Impfstoff subkutan mittels nadellosem Injektor | | | | |
|---|---|---|---|---|
| 1494 | TTK9 | 4096 | 4096 | 8192 |
| 1495 | USK9 | 4096 | 4096 | 8192 |
| 1497 | UVL9 | 4096 | 8192 | 4096 |
| 1499 | TVL9 | 2048 | 2048 | 2048 |

## Patentansprüche

1. Nadelloser Injektor für eine Flüssigkeit, der als Handgerät ausgebildet ist, mit einem Gehäuse und einem Vorratsbehälter für die Flüssigkeit, wobei
• das Gehäuse zwei Teile (1;2), (31;32) umfaßt, die miteinander verbunden und gegeneinander drehbar angeordnet sind, und
• der nadellose Injektor ein Sperrspannwerk mit einem zwischen zwei Anschlägen (17;47) verschiebbaren Sprungstück (3;33;53), das mit einer Auslösevorrichtung versehen ist, sowie einen Hohlkolben (7;37;57) enthält, der im Sprungstück (3;33;53) befestigt ist, und der durch das Sperrspannwerk angetrieben wird, wobei der Hohlkolben innerhalb eines Zylinders (8;38) verschiebbar angeordnet ist und einen einzigen Ventilkörper (12;42) enthält, und am Ende des Zylinders eine Düse (9;39) mit mindestens einer Öffnung (10;40) angebracht ist, und der Raum zwischen der Düse und dem Ventilkörper einen Pumpenraum (13;43) bildet, und
• der Vorratsbehälter (14;44;54) für die Flüssigkeit innerhalb des Gehäuses angeordnet ist und als vom nadellosen Injektor separater Behälter ausgebildet ist und mit dem Ende des Hohlkolbens verbunden ist, das aus dem Zylinder herausragt, und
• die Flüssigkeitsmenge, die beim Herausziehen des Hohlkolbens (7) aus dem Zylinder (8) durch den Hohlkolben hindurch in den Pumpenraum (43) gefördert worden ist, durch den Hub (a) und den Querschnitt des Hohlkolbens bestimmt wird,
• die Lage des Hohlkolbenhubs (a) innerhalb des nadellosen Injektors durch die Position der beiden Anschläge (17;47) bestimmt ist.

2. Nadelloser Injektor nach Anspruch 1, wobei
• die beiden Teile (1;2), (31 ;32) des Gehäuses miteinander lösbar verbunden sind.

3. Nadelloser Injektor nach den Ansprüchen 1 und 2, wobei
• die Düse (9;39) nur eine Öffnung (10;40) mit einem hydraulischen Durchmesser von 10 µm bis 500 µm, bevorzugt von 50 µm bis 150 µm, enthält, und
• die Düsenöffnung (10;40) eine Länge von 50 µm bis 500 µm hat, bevorzugt von 100 µm bis 300 µm.

4. Nadelloser Injektor nach den Ansprüchen 1 und 2, wobei
• die Düse (9;39) mehrere Düsenöffnungen enthält, deren hydraulischer Durchmesser gegebenenfalls unterschiedlich ist.

5. Nadelloser Injektor nach den Ansprüchen 1 und 2, wobei
• die Düse (9; 39) mehrere Düsenöffnungen enthält, deren Längsachsen parallel zueinander verlaufen oder gegeneinander divergent geneigt sind.

6. Nadelloser Injektor nach den Ansprüchen 1 bis 5, wobei
• die Position eines Anschlages und damit der Hohlkolbenhub (a) veränderbar ist.

7. Nadelloser Injektor nach den Ansprüchen 1 bis 6, wobei
• das Sperrspannwerk durch Drehen der beiden Gehäuseteile (1;2), (31;32) gegeneinander von Hand spannbar ist.

8. Nadelloser Injektor nach den Ansprüchen 1 bis 7, wobei
• das Sperrspannwerk über ein Schraub-Schub-Getriebe durch Drehen der beiden Gehäuseteile (1;2),(31;32) gegeneinander spannbar ist.

9. Nadelloser Injektor nach den Ansprüchen 1 bis 8, wobei
• das Sperrspannwerk eine Schraubenfeder (6;36), eine Tellerfeder oder eine Blattfeder als Energiespeicher enthält.

10. Nadelloser Injektor nach den Ansprüchen 1 bis 9, wobei
• die Düse (9;39) aus Metall, Kunststoff, Glas, Silizium oder Edelstein, wie Saphir, Rubin, Korund, besteht.

11. Nadelloser Injektor nach den Anprüchen 1 bis 10, wobei
• der separate Vorratsbehälter (14;44;54) für die Flüssigkeit mittels einer Presspassung (19; 49; 59) mit dem Ende des Hohlköbens lösbar verbunden ist, das aus dem Zylinder herausragt.

12. Nadelloser Injektor nach den Ansprüchen 1 bis 11, wobei
• der separate Vorratsbehälter (14;44;54) für die Flüssigkeit mit dem Hohlkolben (7;37;57) lösbar verbunden ist und mit dem Hub des Hohlkolbens innerhalb des Gehäuses verschiebbar ist.

13. Nadelloser Injektor nach den Ansprüchen 1 bis 12, wobei
• der separate Vorratsbehälter (14;44;54) für die Flüssigkeit als austauschbarer Vorratsbehälter ausgebildet ist, und das Sprungstück (53) zum Aufnehmen des separaten Vorratsbehälters eingerichtet ist.

14. Nadelloser Injektor nach den Ansprüchen 1 und 13, wobei
• der separate Vorratsbehälter (54) für die Flüssigkeit mit dem Hohlkolben (57) und dem Sprungstück (53) lösbar verbunden ist, und
• das Sprungstück (53) mit Schnapphaken (51) versehen ist, die in eine umlaufende Rille (52) im Vorratsbehälter eingreifen.

15. Nadelloser Injektor nach den Ansprüchen 1 bis 14, wobei
• das düsenseitige Ende des nadellosen Injektors mit einer Verschlußkappe (18) versehen ist.

16. Nadelloser Injektor nach den Ansprüchen 1 bis 15, wobei
• vor der dem Pumpenraum zugewandten Seite der Düse ein Filter (11) vorhanden ist.

17. Nadelloser Injektor nach den Ansprüchen 1 bis 16, wobei
• der separate Vorratsbehälter (14;44;54) für die Flüssigkeit mit einem flüssigen Arzneimittel bevorzugt aus der Gruppe der Analgetika, Impfstoffe, Antidiabetika, Hormone, Mittel zur Empfängnisverhütung, Vitamine, Antibiotika, Sedativa, antimikrobielle Substanzen, Aminosäuren, Koronarmittel gefüllt ist.

18. Nadelloser Injektor nach Anspruch 1 bis 3 für eine Flüssigkeit, der als Handgerät ausgebildet ist, mit einem Gehäuse und einem Vorratsbehälter für die Flüssigkeit, wobei
• das Sperrspannwerk eine Schraubenfeder (6;36) enthält, und das Sperrspannwerk über ein Schraub-Schub-Getriebe durch Drehen der beiden Gehäuseteile (1;2), (31;32) gegeneinander spannbar ist, und das Sperrspannwerk mit einer Auslösevorrichtung versehen ist, und
• am Ende des Zylinders eine Düse (9;39) mit einer einzigen Öffnung (10;40) angebracht ist, und
• der Vorratsbehälter (14;44;54) mittels einer Preßpassung (59) mit dem Ende des Hohlkolbens verbunden ist, und der mit dem Sprungstück (53) mittels Schnapphaken (51) verbunden ist, die in eine umlaufende Rille (52) im Vorratsbehälter eingreifen, und
• der separate Vorratsbehälter (14;44;54) mit einem flüssigen Arzneimittel gefüllt ist.

19. Nadelloser Injektor nach Anspruch 18, wobei
• vor der dem Pumpenraum zugewandten Seite der Düse ein Filter (11) vorhanden ist.

20. Verwendung des nadellosen Injektors nach den Ansprüchen 1 bis 19 zum Injizieren einer wirkstoffhaltigen Flüssigkeit in pflanzliches Gewebe.

## Claims

1. A needle-less injector for a liquid, which is in the form of a hand unit, comprising a housing and a supply container for the liquid, wherein
- the housing includes two portions (1; 2), (31; 32) which are connected together and are arranged rotatably relative to each other, and
- the needle-less injector includes a locking stressing mechanism with a sprung portion (3; 33; 53) which is displaceable between two abutments (17; 47) and which is provided with a triggering device, and a hollow plunger (7; 37; 57) which is fixed in the sprung portion (3; 33; 53) and which is driven by the locking stressing mechanism, wherein the hollow plunger is arranged slidably within a cylinder (8; 38) and includes a single valve body (12; 42) and mounted at the end of the cylinder is a nozzle (9; 39) with at least one opening (10; 40) and the space between the nozzle and the valve body forms a pump chamber (13; 43), and
- the supply container (14; 44; 54) for the liquid is arranged within the housing and is in the form of a container separate from the needle-less injector and is connected to the end of the hollow plunger which projects out of the cylinder, and
- the amount of liquid which has been conveyed through the hollow plunger (7) into the pump chamber (43) when the hollow plunger is pulled out of the cylinder (8) is determined by the stroke travel (a) and the cross-section of the hollow plunger, and
- the position of the hollow plunger stroke travel (a) within the needle-less injector is determined by the position of the two abutments (17; 47).

2. A needle-less injector according to claim 1 wherein
- the two portions (1; 2), (31; 32) of the housing are releasably connected together.

3. A needle-less injector according to claims 1 and 2 wherein
- the nozzle (9; 39) includes only one opening (10; 40) of a hydraulic diameter of 10 µm to 500 µm, preferably 50 µm to 150 µm, and
- the nozzle opening (10; 40) is of a length of 50 µm to 500 µm, preferably 100 µm to 300 µm.

4. A needle-less injector according to claims 1 and 2 wherein
- the nozzle (9; 39) includes a plurality of nozzle openings whose hydraulic diameters are possibly different.

5. A needle-less injector according to claims 1 and 2 wherein
- the nozzle (9; 39) includes a plurality of nozzle openings whose longitudinal axes extend in mutually parallel relationship or are inclined divergently relative to each other.

6. A needle-less injector according to claims 1 to 5 wherein
- the position of an abutment and thus the hollow plunger stroke travel (a) is variable.

7. A needle-less injector according to claims 1 to 6 wherein
- the locking stressing mechanism can be stressed by rotating the two housing portions (1; 2), (31; 32) relative to each other by hand.

8. A needle-less injector according to claims 1 to 7 wherein
- the locking stressing mechanism can be stressed by way of a worm-thrust transmission by rotation of the two housing portions (1; 2), (31; 32) relative to each other.

9. A needle-less injector according to claims 1 to 8 wherein
- the locking stressing mechanism includes a coil spring (6; 36), a plate spring or a leaf spring as an energy storage means.

10. A needle-less injector according to claims 1 to 9 wherein
- the nozzle (9; 39) comprises metal, plastic material, glass, silicon or precious stone such as sapphire, ruby or corundum.

11. A needle-less injector according to claims 1 to 10 wherein
- the separate supply container (14; 44; 54) for the liquid is releasably connected by means of a press fit (19; 49; 59) to the end of the hollow plunger which projects out of the cylinder.

12. A needle-less injector according to claims 1 to 11 wherein
- the separate supply container (14; 44; 54) for the liquid is releasably connected to the hollow plunger (7; 37; 57) and is displaceable with the stroke movement of the hollow plunger within the housing.

13. A needle-less injector according to claims 1 to 12 wherein
- the separate supply container (14; 44; 54) for the liquid is in the form of a replaceable supply container and the sprung portion (53) is adapted to receive the separate supply container.

14. A needle-less injector according to claims 1 and 13 wherein
- the separate supply container (54) for the liquid is releasably connected to the hollow plunger (57) and the sprung portion (53), and
- the sprung portion (53) is provided with snap hooks (51) which engage into a peripherally extending groove (52) in the supply container.

15. A needle-less injector according to claims 1 to 14 wherein
- the nozzle end of the nozzle-less injector is provided with a closure cap (18).

16. A needle-less injector according to claims 1 to 15 wherein
- a filter (11) is provided in front of the side of the nozzle, which is towards the pump chamber.

17. A needle-less injector according to claims 1 to 16 wherein
- the separate supply container (14; 44; 54) for the liquid is filled with a liquid drug, preferably from the group of analgesics, vaccines, antidiabetic agents, hormones, contraceptives, vitamins, antibiotics, sedatives, antimicrobial substances, amino acids and coronary agents.

18. A needle-less injector according to claims 1 to 3 for a liquid, which is in the form of a hand unit, comprising a housing and a supply container for the liquid, wherein
- the locking stressing mechanism includes a coil spring (6; 36) and the locking stressing mechanism can be stressed by way of a worm-thrust transmission by rotation of the two housing portions (1; 2), (31, 32) relative to each other, and the locking stressing mechanism is provided with a triggering device, and
- a nozzle (9; 39) having a single opening (10; 40) is mounted at the end of the cylinder, and
- the supply container (14; 44; 54) is connected by means of a press fit (59) to the end of the hollow plunger and is connected to the sprung portion (53) by means of snap hooks (51) which engage into a peripherally extending groove (52) in the supply container, and
- the separate supply container (14; 44; 54) is filled with a liquid drug.

19. A needle-less injector according to claim 18 wherein
- a filter (11) is disposed in front of the side of the nozzle, which is towards the pump chamber.

20. Use of the needle-less injector according to claims 1 to 19 for injecting a liquid containing active substance into vegetable tissue.

## Revendications

1. Injecteur sans aiguille pour un liquide, qui est agencé sous forme d'appareil à main, avec un boîtier et un réservoir pour le liquide, où
• le boîtier comprend deux parties (1 ; 2), (31 ; 32) qui sont liées l'une à l'autre et qui sont disposées de manière à pouvoir tourner l'une par rapport à l'autre, et
• l'injecteur sans aiguille contient une détente de blocage avec une pièce à déclic (3 ; 33 ; 53) déplaçable entre deux butées (17 ; 47) qui est munie d'un dispositif de déclenchement, ainsi qu'un piston creux (7 ; 37 ; 57) qui est fixé dans la pièce à déclic (3 ; 33 ; 53), et qui est entraîné par la détente de blocage, où le piston creux est disposé de manière déplaçable à l'intérieur d'un cylindre (8 ; 38) et contient un seul corps de valve (12 ; 42), et un gicleur (9 ; 39) avec au moins une ouverture (10 ; 40) est disposé à l'extrémité du cylindre, et l'espace entre le gicleur et le corps de valve forme un espace de pompage (13 ; 43), et
• le réservoir (14 ; 44 ; 54) pour le liquide est disposé à l'intérieur du boîtier et est agencé sous forme de récipient séparé de l'injecteur sans aiguille et est relié avec l'extrémité du piston creux qui fait saillie du cylindre, et
• la quantité de liquide qui est transportée dans l'espace de pompage (43) par le biais du piston creux lorsque le piston creux (7) est retiré du cylindre (8) est déterminée par la course (a) et la section transversale du piston creux,
• la position de la course (a) du piston creux à l'intérieur de l'injecteur sans aiguille est déterminée par la position des deux butées (17 ; 47).

2. Injecteur sans aiguille selon la revendication 1 où
• les deux parties (1 ; 2) (31 ; 32) du boîtier sont reliées l'une à l'autre de manière détachable.

3. Injecteur sans aiguille selon les revendications 1 et 2 où
• le gicleur (9 ; 39) ne contient qu'une ouverture (10 ; 40) avec un diamètre hydraulique de 10 µm à 500 µm, de préférence de 50 µm à 150 µm, et
• l'ouverture (10 ; 40) du gicleur a une longueur de 50 µm à 500 µm, de préférence de 100 µm à 300 µm.

4. Injecteur sans aiguille selon les revendications 1 et 2 où
• le gicleur (9 ; 39) contient plusieurs ouvertures de gicleur dont le diamètre hydraulique est éventuellement différent.

5. Injecteur sans aiguille selon les revendications 1 et 2 où
• le gicleur (9 ; 39) contient plusieurs ouvertures de gicleur dont les axes longitudinaux s'étendent parallèlement entre eux ou sont éventuellement inclinés de manière divergente les uns par rapport aux autres.

6. Injecteur sans aiguille selon les revendications 1 à 5 où
• la position d'une butée et donc la course (a) du piston creux sont modifiables.

7. Injecteur sans aiguille selon les revendications 1 à 6 où
• la détente de blocage peut être tendue par rotation des deux parties de boîtier (1 ; 2), (31 ; 32) l'une par rapport à l'autre à la main.

8. Injecteur sans aiguille selon les revendications 1 à 7 où
• la détente de blocage peut être tendue par le biais d'un organe d'entraînement à poussée par vissage par rotation des deux parties de boîtier (1 ; 2), (31 ; 32) l'une par rapport à l'autre.

9. Injecteur sans aiguille selon les revendications 1 à 8 où
• la détente de blocage contient un ressort à boudin (6 ; 36), un ressort à disques ou un ressort à lames comme accumulateur d'énergie.

10. Injecteur sans aiguille selon les revendications 1 à 9 où
• le gicleur (9 ; 39) consiste en métal, matière synthétique, verre, silicium ou pierre précieuse comme le saphir, le rubis, le corindon.

11. Injecteur sans aiguille selon les revendications 1 à 10 où
• le réservoir séparé (14 ; 44 ; 54) pour le liquide est relié de manière séparable avec l'extrémité du piston creux qui fait saillie du cylindre au moyen d'un ajustage serré (19 ; 49 ; 59).

12. Injecteur sans aiguille selon les revendications 1 à 11 où
• le réservoir séparé (14 ; 44 ; 54) pour le liquide est relié de manière détachable avec le piston creux ((7 ; 37 ; 57) et est déplaçable avec la course du piston creux à l'intérieur du boîtier.

13. Injecteur sans aiguille selon les revendications 1 à 12 où
• le réservoir séparé (14 ; 44 ; 54) pour le liquide est agencé sous forme de réservoir remplaçable et la pièce à déclic (53) est disposée pour recevoir le réservoir séparé.

14. Injecteur sans aiguille selon les revendications 1 et 13 où
• le réservoir séparé (54) pour le liquide est relié de manière détachable avec le piston creux (57) et la pièce à déclic (53), et
• la pièce à déclic (53) est munie de crochets d'encliquetage (51) qui pénètrent dans une rainure périphérique (52) dans le réservoir.

15. Injecteur sans aiguille selon les revendications 1 à 14 où
• l'extrémité de l'injecteur sans aiguille du côté du gicleur est munie d'un couvercle (18).

16. Injecteur sans aiguille selon les revendications 1 à 15 où
• un filtre (11) est présent devant le côté du gicleur tourné vers l'espace de pompage.

17. Injecteur sans aiguille selon les revendications 1 à 16 où
• le réservoir séparé (14 ; 44 ; 54) pour le liquide est rempli d'un médicament liquide de préférence du groupe des analgésiques, vaccins, antidiabétiques, hormones, agents contraceptifs, vitamines, antibiotiques, sédatifs, substances antimicrobiennes, aminoacides, agents coronariens.

18. Injecteur sans aiguille selon les revendications 1 à 3 pour un liquide, qui est agencé sous forme d'appareil à main, avec un boîtier et un réservoir pour le liquide, où
• la détente de blocage contient un ressort à boudin (6 ; 36) et la détente de blocage peut être tendue par le biais d'un organe d'entraînement à poussée par vissage par rotation des deux parties de boîtier (1 ; 2), (31 ; 32) l'une par rapport à l'autre, et la détente de blocage est munie d'un dispositif de déclenchement, et
• un gicleur (9 ; 39) avec une seule ouverture (10 ; 40) est disposé à l'extrémité du cylindre, et
• le réservoir (14 ; 44 ; 54) est relié avec l'extrémité du piston creux au moyen d'un ajustage serré (59), et est relié avec la pièce à déclic (53) au moyen de crochets d'encliquetage (51) qui pénètrent dans une rainure périphérique (52) dans le réservoir, et
• le réservoir séparé (14 ; 44 ; 54) est rempli d'un médicament liquide.

19. Injecteur sans aiguille selon la revendication 18 où
• un filtre (11) est présent devant le côté du gicleur tourné vers l'espace de pompage.

20. Utilisation de l'injecteur sans aiguille selon les revendications 1 à 19 pour l'injection d'un liquide contenant un principe actif dans un tissu végétal.
